(19) Europäisches Patentamt
European Patent Office
Office européen des brevets

(11) Veröffentlichungsnummer: **0 001 558**
**A1**

# (12) EUROPÄISCHE PATENTANMELDUNG

(21) Anmeldenummer: 78100939.4

(22) Anmeldetag: 20.09.78

(51) Int. Cl.$^2$: C 07 D 335/04
A 61 K 31/38

(30) Priorität: 13.10.77 DE 2746044

(43) Veröffentlichungstag der Anmeldung:
02.05.79 Patentblatt 79/9

(84) Benannte Vertragsstaaten:
CH DE FR GB NL

(71) Anmelder: Merck Patent Gesellschaft mit beschränkter Haftung
Frankfurter Strasse 250
D-6100 Darmstadt(DE)

(72) Erfinder: Kirchlechner, Richard, Dr.
Im Ritterbruch 15
D-6141 Hähnlein(DE)

## (54) Verfahren zur Herstellung von 6-Thiatetracyclin-derivaten.

(57) Die Erfindung betrifft ein neues Verfahren zur Herstellung von 6-Thiatetracyclinderivaten, dadurch gekennzeichnet, daß man Epimerengemische von 1,4,4a, 5,5a, 6, 11, 12a-Oktahydro-3,12-dihydroxy-1,11-dioxo-6-thia-naphthacen-2-carboxamiden durch Behandeln mit einer Base in sterisch einheitliche 6-Thiatetracyclinderivate mit natürlicher KOnfiguration überführt.

EP 0 001 558 A1

Croydon Printing Company Ltd.

Merck Patent Gesellschaft
mit beschränkter Haftung
D a r m s t a d t

Verfahren zur Herstellung von
6-Thiatetracyclin-derivaten

Die Erfindung betrifft ein neues Verfahren zur Herstellung von 6-Thiatetracyclin-derivaten.

Als "6-Thiatetracyclin" soll hier und im folgenden ein Racemat verstanden werden, das aus 4-Dimethylamino—1,4,4a,5,5a,6,11,12a-oktahydro-3,10,12,12a-tetrahydroxy-1,11-dioxo-6-thia-naphthacen-2-carboxamid mit der in der nachstehenden Formel angegebenen Konfiguration:

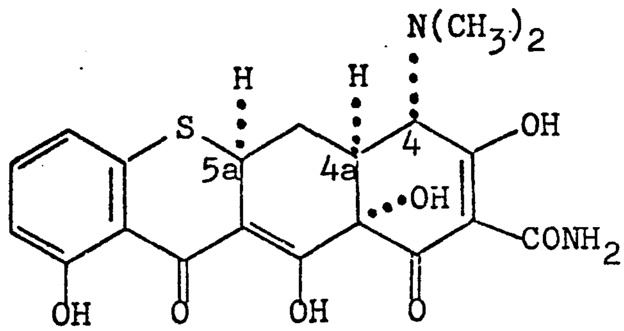

und seinen optischen Antipoden besteht.

Eine Konfiguration, bei der die Aminogruppe an $C_{(4)}$ und die beiden Wasserstoffatome an $C_{(4a)}$ und $C_{(5a)}$ zueinander in syn-Stellung stehen (wie in 6-Thiatetracyclin), wird nachstehend als "natürlich" bezeichnet, da sie derjenigen der natürlichen, durch Mikroorganismen hergestellten Tetracycline entspricht.

6-Thiatetracyclin-derivate sind bekannt, z.B. aus der DT-OS 24 37 487 und der DT-OS 24 42 829. Bei den dort beschriebenen Synthesen kann das tetracyclische System (Oktahydro-6-thianaphthacen) gebildet werden durch Kondensation eines 2-Phenyl-4-[2-(5-hydroxy-thiochroman-4-on-2-yl)-äthyliden]-2-thiazolin-5-ons (oder -oxazolin-5-ons) mit Acetondicarbonsäure-monomethylester-monoamid. Dabei fällt ein Gemisch der 4 möglichen Racemate von 1,4,4a,5,5a,6,11,12a-Oktahydro-3,12-dihydroxy-1,11-dioxo-6-thianaphthacen-2-carboxamiden I bis IV an, von denen die entsprechenden "4aα"-Antipoden im folgenden schematisch angeführt sind:

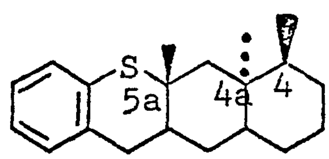

I

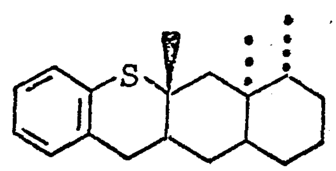

II

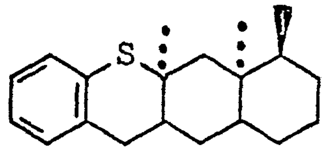

III

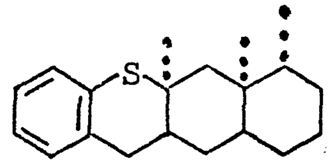

IV

Dabei entspricht IV der "natürlichen" Konfiguration. Verbindungen mit dieser Konfiguration zeigen die größte antibakterielle Wirksamkeit und sind daher bevorzugt. Man wird daher bestrebt sein, die den weniger wirksamen Reihen zugehörigen Isomeren I, II und III in das Isomere IV, das der wirksamsten Reihe zugehört, umzuwandeln.

So kann man (vgl. l.c.) den Substituenten in 4-Stellung im gewünschten Sinne epimerisieren, beispielsweise durch Stehenlassen in Pyridin oder Piperidin; auf diese Weise kann I in II und III in IV umgewandelt werden. Ausgehend von dem Rohprodukt der genannten Kondensation erhält man so ein Gemisch, das nur aus II und IV besteht.

Bisher war jedoch kein Weg bekannt, das weniger erwünschte II in das wertvolle IV umzuwandeln. In der Regel mußte also das erhaltene Gemisch aufgetrennt werden, was wegen der chemischen Ähnlichkeit der beiden Komponenten mühsam ist und beispielsweise mit Hilfe chromatographischer Methoden gelang. Das Isomere II mußte in der Regel verworfen werden, da es nicht in Folgeprodukte umgewandelt werden konnte, die in vivo pharmakologisch wirksam waren.

Der Erfindung lag die Aufgabe zugrunde, ein Verfahren aufzufinden, mit dessen Hilfe Epimerengemische, die mindestens zwei der Racemate I bis IV enthalten, in reines Racemat IV übergeführt werden können. Diese Aufgabe wurde durch die Bereitstellung des erfindungsgemäßen neuen Verfahrens gelöst.

Es wurde nun gefunden, daß es wider Erwarten doch gelingt, II in IV umzuwandeln, und zwar durch Behandeln mit einer Base bei erhöhter Temperatur und/oder längeren Reaktionszeiten.

Als Basen eignen sich insbesondere gesättigte heterocyclische sekundäre Amine mit insgesamt 4 - 12, vorzugsweise 4 - 9 C-Atomen, insbesondere Piperidin, ferner z.B. Pyrrolidin oder Morpholin.

Man kann in Anwesenheit oder Abwesenheit eines zusätzlichen inerten Lösungsmittels arbeiten. Als Lösungsmittel eignen sich beispielsweise Amide, vorzugsweise Dimethylformamid (DMF), ferner Diäthylformamid, Dimethylacetamid, Phosphorsäure-hexamethyltriamid; Sulfoxide wie Dimethylsulfoxid.

Man arbeitet zweckmäßig bei Temperaturen zwischen etwa 15 und 120°, vorzugsweise zwischen 40 und 60°. Bei einer Reaktionstemperatur von etwa 50° ist die Umwandlung nach etwa einer Stunde beendet, bei 20° nach etwa 12 Stunden. Sie gelingt mit hohen Ausbeuten, praktisch quantitativ, so daß die Aufarbeitung des Reaktionsgemischs sehr vereinfacht wird, da nur ein Isomeres vorliegt, und zwar von den möglichen vier Isomeren allein das gewünschte Produkt.

Da durch die Einwirkung der Base auch eine Epimerisierung des Substituenten in 4-Stellung bewirkt wird, kann man das Rohprodukt des Ringschlusses, das alle vier Isomeren enthält, direkt in die Umlagerungsreaktion einsetzen. Dabei laufen die oben erwähnten Epimerisierungen von I zu II und von III zu IV und die Umlagerung von II zu IV im "Eintopfverfahren" ab. So können mit Hilfe des neuen Verfahrens nicht nur II und II enthaltende Gemische, sondern schlechthin IV entsprechende Epimerengemische, insbesondere IV entsprechende 5a-Epimerengemische, in IV übergeführt werden.

Gegenstand der Erfindung ist dementsprechend ein Verfahren zur Herstellung von 6-Thiatetracyclinderivaten dadurch gekennzeichnet, daß man Epimerengemische von 1,4,4a,5,5a,6,11,12a-Oktahydro-3,12,dihydroxy-1,11-dioxo-6-thia-naphthacen-2-carboxamiden durch Behandeln mit einer Base in sterisch einheitliche 6-Thiatetracyclinderivate mit natürlicher Konfiguration überführt.

Die Umwandlung des festgefügten Ringsystems kann nur über eine intermediäre Ringöffnung erklärt werden:

Unter den Reaktionsbedingungen öffnet sich der Ring C von II in einer Retro-Michael-Reaktion zum - nicht isolierten - Zwischenprodukt V und schließt sich in einer Michael-Reaktion dann wieder zu IV, das anscheinend das thermodynamisch stabilere Produkt ist:

II 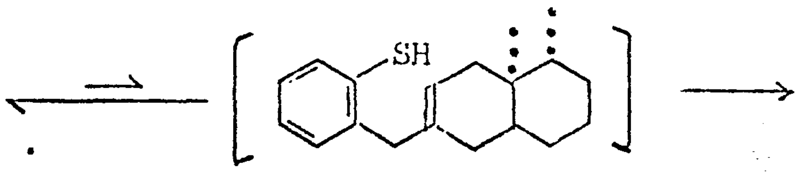 IV

V

Wenn auch das Gleichgewicht zwischen II und V stark auf der Seite von II liegen dürfte, gelingt es doch, praktisch das gesamte II in IV umzuwandeln.

Vorzugsweise sind erfindungsgemäß 12α-Deshydroxy-6-thiatetracycline der allgemeinen Formel VII erhältlich:

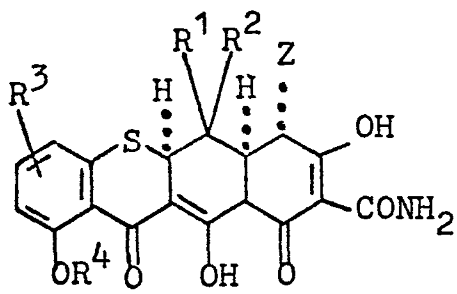

VII

worin

| | |
|---|---|
| $R^1$, $R^2$ und $R^4$ | jeweils H oder Alkyl, |
| $R^3$ | H, F, Cl, Br, $CF_3$, OH, Alkyl, Alkoxy, $NO_2$, $NH_2$, Alkylamino, Dialkylamino oder Acylamino und |
| Z | eine funktionell abgewandelte Aminogruppe |

bedeuten,
wobei die Alkyl- und Alkoxygruppen jeweils 1 bis 3, die Acylgruppen jeweils 1 bis 4 C-Atome besitzen.

In den Verbindungen der Formel VII ist die 4-Aminogruppe vorzugsweise in Form eines Acyl- oder Thioacylderivats oder eines davon abgeleiteten Iminoäthers oder Iminothioäthers abgewandelt. Sie liegt dementsprechend vorzugsweise in Form einer der Gruppen $-NR^5-CY-R^6$ oder $-N=C(YR^7)-R^6$ vor, worin $R^5$ H oder Alkyl, $R^6$ insbesondere H, Alkyl mit 1 - 10 C-Atomen oder einen unsubstituierten oder durch Alkyl mit 1 - 4 C-Atomen, OH, intermediär geschütztes OH, $CH_2OH$ mit gegebenenfalls intermediär geschützter OH-Gruppe, $NO_2$, $NH_2$, Alkylamino, Dialkylamino, Hydroyalkylamino, Acylamino, Halogen, COOH, COOAlkyl, $CONH_2$ und/oder CONHAlkyl mono- oder disubstituierten Phenyl-, Benzyl-, Phenoxymethyl- oder Phenoxypropylrest, die Gruppe Y ein Sauerstoff- oder ein Schwefelatom und $R^7$ Alkyl bedeuten, wobei die Alkylgruppen vorzugsweise bis zu 4, die Acylgruppen vorzugsweise bis zu 7-C-Atome enthalten. Bevorzugt ist Z eine $C_6H_5-CY-NH$-Gruppe.

Die erfindungsgemäß erhältlichen Verbindungen können als Arzneimittel in der Human- und Veterinärmedizin oder als Zwischenprodukte zur Herstellung anderer Arzneimittel verwendet werden. So können die Verfahrensprodukte, insbesondere diejenigen der Formel VII, nach bekannten Methoden (vgl. l.c.) in andere pharmakologisch wirksame 6-Thiatetracyclinderivate umgewandelt werden. So ist z.B. das 6-Thiatetracyclin selbst mit Hilfe des vorliegenden Verfahrens besonders rationell und in guter Gesamtausbeute erhältlich.

Beispiel 1

Man löst 10,9 g 2-Phenyl-4-/2-(5-hydroxy-thiochroman-4-on-2-yl)-äthyliden/-2-thiazolin-5-on (F. 144°) und 5,8 g Acetondicarbonsäure-monomethylester-monoamid unter Stickstoff in 100 ml Dioxan, versetzt mit 0,87 g Natriumhydrid (80 %ig) und rührt eine Stunde bei 20°. Dann gibt man weitere 2,6 g Natriumhydrid hinzu, rührt 10 Minuten, versetzt mit 100 ml DMF und kocht eine Stunde. Man kühlt ab, zersetzt überschüssiges Natriumhydrid mit Methanol und gießt unter Rühren in ein Gemisch aus Eis und Salzsäure. Das ausgefallene Gemisch ("G") von stereoisomeren 4-Thiobenzamido-1,4,4a,5,5a,6,11,12a-oktahydro-3,10,12-trihydroxy-1,11-dioxo-6-thianaphthacen-2-carboxamiden wird abfiltriert, mit Wasser gewaschen, getrocknet und unter Stickstoff in einem Gemisch von 75 ml Piperidin und 75 ml DMF gelöst. Man erwärmt eine Stunde auf 50°, kühlt ab und rührt in ein Gemisch aus Salzsäure und Eis. Das erhaltene reine 4-Desdimethylamino-4-thiobenzamido-12a-des-hydroxy-6-thiatetracyclin ("D") wird abfiltriert, mit Wasser gewaschen, getrocknet und aus Aceton umkristallisiert, F. 195 - 196°.

Das Ausgangsmaterial ist wie folgt erhältlich:

5-Hydroxy-8-chlor-thiochroman-4-on-2-acetonitril (vgl. DT-OS 24 42 829) wird zur Säure (F. 141 - 143°) hydrolysiert; Hydrogenolyse liefert 5-Hydroxy-thiochroman-4-on-2-essigsäure (F. 160 - 164°), die über das harzige Chlorid nach der Methode von Rosenmund zum Aldehyd (F. 69°) reduziert wird; dieser wird mit 2-Phenyl-2-thiazolin-5-on kondensiert.

Beispiel 2

Man löst 21,2 g 2-Phenyl-4-/2-(5-hydroxy-8-dimethylamino-thiochroman-4-on-2-yl)-äthyliden7-2-thiazolin-5-on. F. 148 - 150°; erhältlich durch Ätherspaltung von 5-Methoxy-8-nitro-thiochroman-4-on-2-essigsäure (vgl. DT-OS 24 42 829) zu 5-Hydroxy-8-nitro-thiochroman-4-on-2-essigsäure (F. 182°), Reduktion und anschließende Methylierung zu 5-Hydroxy-8-dimethylamino-thiochroman-2-essigsäure (F. 150 - 152°) Überführung in das Chlorid, Reduktion zum Aldehyd (ölig) und Kondensation mit 2-Phenyl-2-thiazolin-5-on7 und 8,75 g Acetondicarbonsäure-monomethylester-monoamid unter Stickstoff in 200 ml Pyridin, versetzt mit 0,5 g NaH und rührt über Nacht bei 20°. Dann gibt man weitere 4,9 NaH hinzu, kocht 1,5 Stunden, kühlt ab, rührt in ein Gemisch aus Eis und Salzsäure und gibt Natronlauge bis pH 3 - 4 hinzu. Das ausgefallene Gemisch von stereoisomeren 4-Thiobenzamido-7-dimethyl-amino-1,4,4a,5,5a,6,11,12a-okta-hydro-3,10,12-trihydroxy-1,11-dioxo-6-thianaphthacen-2-carboxamiden wird abfiltriert, mit Wasser gewaschen, getrocknet und in einem Gemisch aus 125 ml Piperidin und 125 ml DMF gelöst. Man erwärmt 75 Minuten auf 50°, kühlt ab und rührt in ein Gemisch aus Salzsäure und Eis. Nach Zugabe von Natronlauge bis pH 3 - 4 saugt man das erhaltene reine 4-Des-Dimethylamino-4-thiobenzamido-7-dimethylamino-12a-des-hydroxy-6-thia-tetracyclin ab, wäscht mit Wasser, trocknet und kristallisiert aus Methylenchlorid um. F. 222 - 223°.

Analog erhält man:

4-Des-dimethylamino-4-thiobenzamido-7-methoxy-12a-des-hydroxy-6-thiatetracyclin, F. 253 - 254°;
4-Des-dimethylamino-4-thiobenzamido-7-chlor-12a-des-hydroxy-6-thiatetracyclin, F. 255°;
4-Des-dimethylamino-4-thiobenzamido-8-methoxy-12a-des-hydroxy-6-thiatetracyclin, F. 212 - 215°.

Beispiel 3

Man löst 1 g Gemisch "G" (vgl. Beispiel 1) in 5 ml Piperidin und läßt über Nacht bei 20° stehen. Nach Aufarbeitung analog Beispiel 1 erhält man reines "D", F. 195 - 196°.

Beispiel 4

Man löst 1 g Gemisch "G" in 5 ml Morpholin, erwärmt 1 Stunde auf 50°, arbeitet analog Beispiel 1 auf und erhält reines "D", F. 195 - 196°.

Beispiel 5

Man löst 1 g "G" in 10 ml Pyrrolidin, erhitzt 10 Minuten auf 70°, kühlt ab, arbeitet analog Beispiel 1 auf und erhält reines "D", F. 195 - 196°.

Merck Patent Gesellschaft
mit beschränkter Haftung
D a r m s t a d t

## Patentanspruch

Verfahren zur Herstellung von 6-Thiatetracyclin-derivaten, dadurch gekennzeichnet, daß man Epimerengemische von 1,4,4a,5,5a,6,11,12a-Oktahydro-3,12-dihydroxy-1,11-dioxo-6-thia-naphthacen-2-carboxamiden durch Behandeln mit einer Base in sterisch einheitliche 6-Thiatetracyclinderivate mit natürlicher Konfiguration überführt.

Europäisches Patentamt

# EUROPÄISCHER RECHERCHENBERICHT

Nummer der Anmeldung
EP 78 100 939.4

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | DE - A - 2 442 829 (MERCK PATENT)<br>* Seite 14 oben *<br>-- | 1 |
| DA | DE - A - 2 437 487 (MERCK PATENT)<br>* ganzes Dokument *<br>---- | |

KLASSIFIKATION DER ANMELDUNG (Int.Cl.²)

C 07 D 335/04
A 61 K 31/38

RECHERCHIERTE SACHGEBIETE (Int. Cl.²)

A 61 K 31/38
C 07 D 335/04

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 16-01-1979 | FROELICH |